# EUROPEAN PATENT SPECIFICATION

(11) **EP 2 892 024 B1**
(45) Date of publication and mention of the grant of the patent: **18.07.2018**
(21) Application number: 14167888.8
(22) Date of filing: 12.05.2014
(51) Int. Cl.: G06T 3/60

(54) **Method and medical imaging apparatus for displaying medical images**
Verfahren und medizinische Bildgebungsvorrichtung zur Anzeige medizinischer Bilder
Procédé et appareil d'imagerie médicale pour afficher des images médicales

(30) Priority: 07.01.2014 KR 20140002078
(43) Date of publication of application: 08.07.2015
(73) Proprietor: Samsung Medison Co., Ltd., Gangwon-do 250-870 (KR)
(72) Inventor: Shim, Eun-a, Gyeonggi-do (KR); Kim, Sung-yoon, Gyeonggi-do (KR); Kim, Chul-an, Gyeonggi-do (KR)
(74) Representative: Schmid, Wolfgang

(56) References cited:
- WO-A2-2007/110420
- VON KAISENBERG C S ET AL: "Fetal transabdominal anatomy scanning using standard views at 11 to 14 weeks' gestation", AMERICAN JOURNAL OF OBSTETRICS & GYNECOLOGY, MOSBY, ST LOUIS, MO, US, vol. 192, no. 2, 1 February 2005 (2005-02-01), pages 535-542, XP004732357,
- WHITLOW BJ ET AL.: "The Effect of Fetal Neck Position on Nuchal Translucency Measurement", BRITISH JOURNAL OF OBSTETRICS AND GYNAECOLOGY, vol. 105, August 1998 (1998-08), pages 872-876, XP002740572,
- EDWARDS A. ET AL.: "The Effect of Image Size on Nuchal Translucency Measurement", PRENATAL DIAGNOSIS, 2003, pages 284-286, XP002740573,
- Thomas Schiemann ET AL: "Interpretation of Tomographic Images Using Automatic Atlas Lookup", Proc. SPIE 2359, Visualization in Biomedical Computing 1994, 9 September 1994 (1994-09-09), pages 457-465, XP55271432, Retrieved from the Internet: URL:https://www.google.de/url?sa=t&rct=j&q =&esrc=s&source=web&cd=1&ved=0ahUKEwiHk82j oM7UAhUHblAKHaPXCsEQFggpMAA&url=http%3A%2F %2Fproceedings.spiedigitallibrary.org%2Fpd faccess.ashx%3Furl%3D%2Fdata%2Fconferences %2Fspiep%2F51452%2F457_1.pdf&usg=AFQjCNFVD swudMLCKlt6CB-Q6-CSqQrBCg&cad=rja [retrieved on 2016-05-10]
- COLLINS D LOUIS ET AL: "Automatic 3D Intersubject Registration of MR Volumetric Data in Standardized Talairach Space", JOURNAL OF COMPUTER ASSISTED TOMOGRAPHY, LIPPINCOTT WILLIAMS AND WILKINS, US, vol. 18, no. 2, 1 March 1994 (1994-03-01), pages 192-205, XP009178279, ISSN: 0363-8715

## Description

### BACKGROUND

### 1. Field

One or more embodiments of the present invention relate to a method and medical imaging apparatus for displaying a medical image, and more particularly, to technologies for displaying medical images that are suitable for a standard view.

### 2. Description of the Related Art

Various types of medical imaging devices are used to observe the internal structures of a human body and diagnose diseases. Examples of medical imaging devices may include a magnetic resonance imaging (MRI) device, a computed tomography (CT) device, an ultrasound diagnosis device, an X-ray system, and a positron emission tomography (PET) device.

In order to make a diagnosis based on a medical image acquired by a medical imaging device, it is necessary to change a geometry of the medical image to one appropriate for a standard view.

For example, fetal images contained in a mid-sagittal plane (MSP) image may need to meet several requirements to measure fetal nuchal translucency (NT) in an ultrasound image. Information related to a standard view that is a protocol for measurement of fetal NT is described in http://www.fetalmedicine.com/fmf/training-certification/certificates-of-competence/11-13-week-scan/nuchal/. In order for an MSP image to satisfy the above-described requirements, users have suffered the inconvenience of having to enlarge, reduce, translate, or rotate the MSP image.

Von Kaisenberg C S et al: "Fetal transabdominal anatomy scanning using standard views at 11 to 14 weeks' gestation", American Journal of Obstetrics & Gynecology, Mosby, St. Louis Mo, US, vol. 192, no. 2, 1 February 2005 (2005-02-01), pages 535-542, XP004732357 shows that transabdominal fetal anatomy scanning with standard fetal anatomy views at 11 to 14 weeks of gestation is possible with good reproducibility and demonstrability when harmonic and compound imaging are used.

Edwards A. et al.: "The Effect of Image Size on Nuchal Translucency Measurement", Prenatal Diagnosis, 2003, pages 284-286 discloses that the measurement of NT decreases significantly with increasing image size. Optimization of NT as a method of screening will require agreed standardization of image magnification.

The WO 2007/110420 A2 describes methods for fully automatic quantification and interpretation of three dimensional images of the brain or other organs. A system for Computer Aided Diagnosis (CAD) of diseases affecting cerebral cortex from SPECT images of the brain, where said images may represent cerebral blood flow (CBF). The methods include image processing, statistical shape models, a virtual brain atlas, reference databases and machine learning.

### SUMMARY

One or more embodiments of the present invention include a method and medical imaging apparatus that are capable of displaying a view of a medical image of which a geometry has been transformed so that the medical image is suitable for a standard view.

Additional aspects will be set forth in part in the description which follows and, in part, will be apparent from the description, or may be learned by practice of the presented embodiments.

The invention is defined by the method of claim 1.

The view of the medical image is an image that is obtained by performing at least one of enlargement, reduction, rotation, and shift on the medical image and displayed on the display unit.

The standard view includes information about at least one of a position, a size, and a direction of a structure that should be included in the view of the medical image.

In the displaying of the view of the medical image, a fitness rate between the standard view and the view of the medical image may be displayed.

The at least one landmark point may include positions of two landmark points. The view of the medical image that satisfies the standard view may include an image obtained by enlarging or reducing the medical image based on a ratio between a distance between the two landmark points and an overall size of the view of the medical image to be displayed through the display unit and a ratio included in the standard view.

The view of the medical image that satisfies the standard view may include an image obtained by shifting the medical image so that the at least one landmark point is located at a position included in the standard view.

The view of the medical image that satisfies the standard view may include an image obtained by rotating the medical image at an angle determined based on the at least one landmark point and the standard view.

The method may further include acquiring seed information related to the medical image.In the acquiring of the information about the at least one landmark point, the information about the at least one landmark point may be acquired based on the seed information.

The medical image is an ultrasound image.

The method further includes selecting a diagnostic mode,
wherein the requirements included in the standard view are determined according to a selected diagnostic mode.

Regarding the medical imaging apparatus the invention is defined by claim 6.

The view of the medical image is an image that is obtained by performing at least one of enlargement, reduction, rotation, and shift on the medical image and displayed on the display unit.

The standard view includes information about at least one of a position, a size, and a direction of a structure that should be included in the view of the medical image.

The display unit may display a fitness rate between the standard view and the view of the medical image.

The at least one landmark point may include positions of two landmark points. The view of the medical image that satisfies the standard view may include an image obtained by enlarging or reducing the medical image based on a ratio between a distance between the two landmark points and an overall size of the view of the medical image to be displayed through the display unit and a ratio included in the standard view.

The view of the medical image that satisfies the standard view may include an image obtained by shifting the medical image so that the at least one landmark point is located at a position included in the standard view.

The view of the medical image that satisfies the standard view may include an image obtained by rotating the medical image at an angle determined based on the at least one landmark point and the standard view.

The information acquisition unit may further acquire seed information related to the medical image and information about the at least one landmark point based on the seed information.

The medical image is an ultrasound image.

The apparatus further includes a diagnostic mode selector for selecting a diagnostic mode. The display unit may display the view of the medical image that satisfies requirements included in the standard view that are determined according to a selected diagnostic mode.

According to one or more embodiments of the present invention, a non-transitory computer-readable recording medium has recorded thereon a program for executing the above-described method on a computer.

### BRIEF DESCRIPTION OF THE DRAWINGS

These and/or other aspects will become apparent and more readily appreciated from the following description of the embodiments, taken in conjunction with the accompanying drawings in which:
FIG. 1 is a diagram of a structure of a medical imaging apparatus according an exemplary embodiment of the present invention;
FIG. 2 is a flowchart of a process of displaying a medical image, according to an exemplary embodiment of the present invention;
FIG. 3 is a conceptual diagram of a standard view according to an exemplary embodiment of the present invention;
FIG. 4 is a conceptual diagram illustrating a medical image view according to an exemplary embodiment of the present invention;
FIG. 5 is a conceptual diagram illustrating a process of enlarging a medical image, which is performed by a medical imaging apparatus, according to an exemplary embodiment of the present invention;
FIG. 6 is a conceptual diagram of a process of shifting a medical image, which is performed by a medical imaging apparatus, according to an exemplary embodiment of the present invention;
FIG. 7 is a conceptual diagram of a process of rotating a medical image, which is performed by a medical imaging apparatus, according to an exemplary embodiment of the present invention;
FIG. 8 is a conceptual diagram showing measurement of medical information from a medical image displayed in a view, according to an exemplary embodiment of the present invention; and
FIG. 9 is a block diagram of a configuration of a medical imaging apparatus implemented as an ultrasound diagnostic device, according to an exemplary embodiment of the present invention.

### DETAILED DESCRIPTION

Exemplary embodiments of the present invention will now be described more fully hereinafter with reference to the accompanying drawings so that they may be easily implemented by one of ordinary skill in the art. However, the present embodiments may have different forms and should not be construed as being limited to the descriptions set forth herein. In addition, parts not related to the present invention are omitted to clarify the description of exemplary embodiments of the present invention. In the accompanying drawings, like reference numerals refer to like elements throughout.

Throughout the specification, it will be understood that when an element is referred to as being "connected" or "coupled" to another element, it can be directly connected to or electrically coupled to the other element with one or more intervening elements interposed therebetween. Expressions such as "at least one of," when preceding a list of elements, modify the entire list of elements and do not modify the individual elements of the list.

Throughout the specification, it will also be understood that when a part "includes" or "comprises" an element, unless there is a particular description contrary thereto, the part can further include other elements, not excluding the other elements. In addition, terms such as "... unit", "... module", or the like refer to units that perform at least one function or operation, and the units may be implemented as hardware or software or as a combination of hardware and software.

Throughout the specification, an "ultrasonic image" refers to an image of an object obtained using an ultrasonic wave. Furthermore, in the present specification, an "object" may include a person or an animal, or a part of a person or an animal. For example, the object may include the liver, the heart, the womb, the brain, a breast, the abdomen, or a blood vessel. Furthermore, the "object" may include a phantom. The phantom means a material having a volume that is approximately the intensity and effective atomic number of a living organism.

Furthermore, in the present specification, a "user" refers to a medical professional, such as a doctor, a nurse, a medical laboratory technologist, a medical imaging expert, and a technician who repairs a medical apparatus, but the user is not limited thereto.

For convenience of explanation, embodiments of the present invention provide a method and apparatus for displaying an ultrasound image according to a standard view for measuring fetal nuchal translucency (NT) in an ultrasound image. However, the present invention is not limited thereto, and it will be apparent to those of ordinary skill in the art that the present invention can be applied to other types of medical images.

The term "landmark point" as used herein may mean a point or region in an image which indicates a feature.

The embodiments of the present invention will now be described in detail with reference to the accompanying drawings.

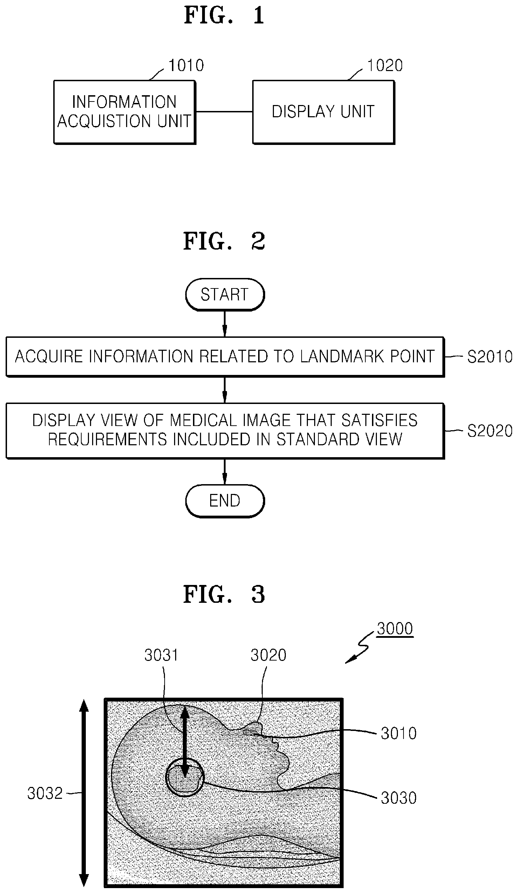

FIG. 1 is a diagram of a structure of a medical imaging apparatus according to an exemplary embodiment of the present invention. The medical imaging apparatus according to the present embodiment includes an information acquisition unit 1010 and a display unit 1020.

The information acquisition unit 1010 acquires information related to at least one landmark point in a medical image. Here, a landmark point means a point indicating a feature related to a body structure contained in a medical image. The information related to the at least one landmark point may include information such as a position of a landmark point. Alternatively, the information related to the at least one landmark point may further include a distance between landmark points and an area related to a body structure corresponding to a landmark point.

For example, a distance between thalamus or diencephalon and a head outline in a fetal ultrasound image may be required to capture a mid-sagittal plane (MSP) image in the fetal ultrasound image. The distance may be input from a user, or positions of the thalamus or diencephalon and the head outline may be detected using an image recognition technique. In this case, the medical imaging apparatus may acquire the positions of the thalamus or diencephalon and the head outline as seed information necessary for capturing an MSP image. The information acquisition unit 1010 may acquire information related to a landmark point based on the seed information.

In one embodiment, the display unit 1020 may display a view of a medical image that satisfies requirements included in a standard view, based on a landmark point.

The standard view includes requirements that have to be met by a medical image to be displayed on a medical imaging apparatus for diagnosis based on the medical image. The standard view may include information about at least one of a position, a size, and a direction of a structure. FIG. 3 is a conceptual diagram of a standard view 3000 for measuring fetal NT, according to an exemplary embodiment of the present invention. Referring to FIG. 3, requirements for positions of a nasal bone 3010, a nasal tip 3020, and diencephalon 3030, and a ratio between a distance 3031 from the diencephalon 3030 to a head outline and an overall size 3032 of a view may be defined according to the standard view 3000. However, the present invention is not limited thereto, and the overall size 3032 of the view that is displayed on a medical imaging apparatus may be determined according to a length, a width, a length of a diagonal line, or an area of the view.

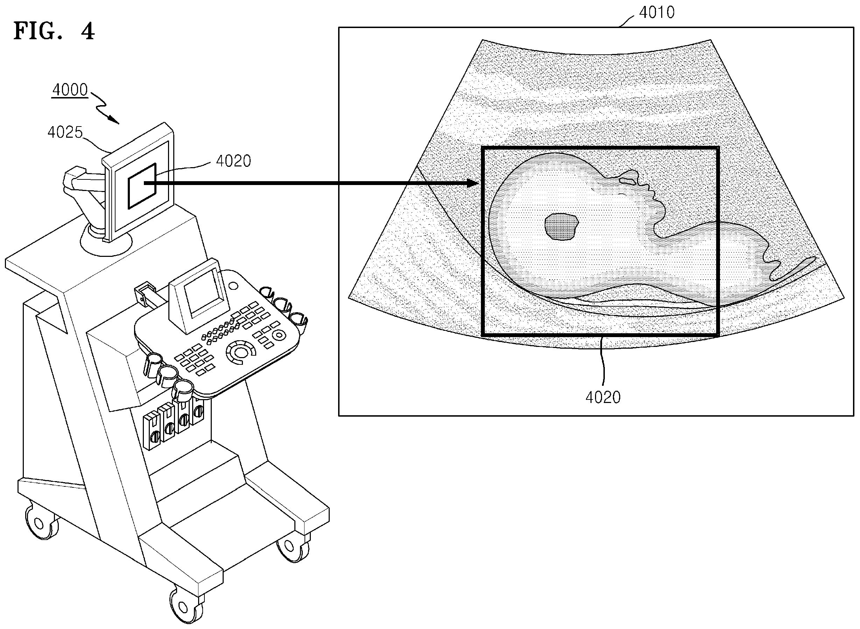

Furthermore, a medical image view is a region on a medical imaging apparatus where a medical image is displayed. FIG. 4 is a conceptual diagram illustrating a medical image view 4020 according to an exemplary embodiment of the present invention. Referring to FIG. 4, a display device 4000 according to an embodiment of the present invention may include a display unit 4025 for displaying an image. The display unit 4025 may include various types of devices for outputting an image. For example, the display unit 4025 may include a liquid crystal display (LCD), a plasma display panel (PDP), or a cathode ray tube (CRT) display. The display unit 4025 is configured to display the medical image view 4020 representing at least a part of the medical image 4010. In other words, the display unit 4025 may display a portion of the medical image 4010 included in the medical image view 4020.

Referring back to FIG. 1, the display unit 1020 may display a medical image view including a medical image, which satisfies requirements included in a standard view, by using a relationship between the medical image view and features of an object including a size of a structure in the medical image and seed information. In this case, the display unit 1020 may perform at least one of enlargement, reduction, rotation, and translation on the medical image so that the medical image in the medical image view satisfies requirements included in a standard view.

In another embodiment, the display unit 1020 may also display a fitness rate between a standard view and a medical image view. The fitness rate may be represented in a qualitative or quantitative manner. For example, the fitness rate may be expressed qualitatively as "excellent", "good", and "poor", or "success" and "failure". As another example, the fitness rate may be expressed quantitatively as "100%/75%/50%/25%/0%" or "5/4/3/2/1" according to the number of requirements that a medical image view displayed on the display unit 1020 satisfies among requirements included in a standard view. In another embodiment, if the fitness rate is classified qualitatively as "poor" or "failure" or does not exceed a quantitative thread value, the display unit 1020 may display a message indicating readjustment of the medical image view.

The medical imaging apparatus further includes a diagnostic mode selector (not shown) for selecting a diagnostic mode. In this case, the display unit 1020 may display a view of a medical image of which a geometry has been transformed based on a standard view determined according to a selected diagnostic mode. For example, an ultrasound diagnostic device may select a diagnostic mode for measuring fetal NT from among a plurality of diagnostic modes according to a user's input to the ultrasound diagnostic device. When the diagnostic mode for measuring fetal NT is selected, a display unit of the ultrasound diagnostic device may change a geometry of an ultrasound image into one suitable for a standard view for measuring fetal NT and display an ultrasound image of which the geometry has been changed.

FIG. 2 is a flowchart of a process of displaying a medical image, according to an exemplary embodiment of the present invention.

Referring to FIG. 2, first, an information acquisition unit of a medical imaging apparatus may acquire information related to at least one landmark point (S2010). Here, the landmark point means a point indicating a feature related to a body structure in a medical image. The information related to the at least one landmark point may include information such as a position of the landmark point. Alternatively, the information related to the at least one landmark point may further include a distance between landmark points and an area related to the body structure corresponding to the landmark point.

The process of displaying a medical image further includes an operation (not shown) of acquiring seed information related to the medical image. The operation may be performed by the information acquisition unit. In this case, the information acquisition unit may acquire information related to the at least one landmark point based on the seed information in operation S2010.

For example, a distance between thalamus or diencephalon and a head outline in a fetal ultrasound image may be required to capture an MSP image in the fetal ultrasound image. The distance may be input from a user, or positions of the thalamus or diencephalon and the head outline may be detected. In this case, the medical imaging apparatus may acquire the positions of the thalamus or diencephalon and the head outline as seed information necessary for capturing the MSP image. In this way, the information acquisition unit may acquire information related to the at least one landmark point based on the seed information.

Thereafter, a display unit of the medical imaging apparatus may display a view of a medical image. The view satisfies requirements included in a standard view (S2020).

In this case, the standard view includes requirements that have to be met by the view of a medical image to be displayed on the medical imaging apparatus for diagnosis based on the medical image. The standard view may include information about at least one of a position, a size, and a direction of a structure that should be included in the view of the medical image. As described above with reference to FIG. 3 requirements for positions of the nasal bone 3010, the nasal tip 3020, and the diencephalon 3030, and a ratio between the distance 3031 from the diencephalon 3030 to the head outline and the overall size 3032 of a view may be defined according to the standard view 3000. However, the present invention is not limited thereto, and the overall size 3032 of the view that is displayed on the medical imaging apparatus may be determined according to a length, a width, a length of a diagonal line, or an area of the view.

Furthermore, the medical image view is a region on the medical imaging apparatus where the medical image is displayed. As described above with reference to FIG. 4, the display device 4000 according to the present embodiment may include the display unit 4025 for displaying an image. The display unit 4025 may include various types of devices for outputting an image. For example, the display unit 4025 may include an LCD, a PDP, or a CRT display. The display unit 4025 is configured to display the medical image view 4020 representing at least a part of the medical image 4010. In other words, the display unit 4025 may display a portion of the medical image 4010 included in the medical image view 4020.

Referring back to FIG. 2, the display unit may display the medical image view including the medical image, which satisfies requirements included in the standard view, by using a relationship between the medical image view and features of an object including a size of a structure in the medical image and seed information. In this case, the display unit may perform at least one of enlargement, reduction, rotation, and translation on the medical image so that the medical image in the medical image view satisfies the requirements included in the standard view.

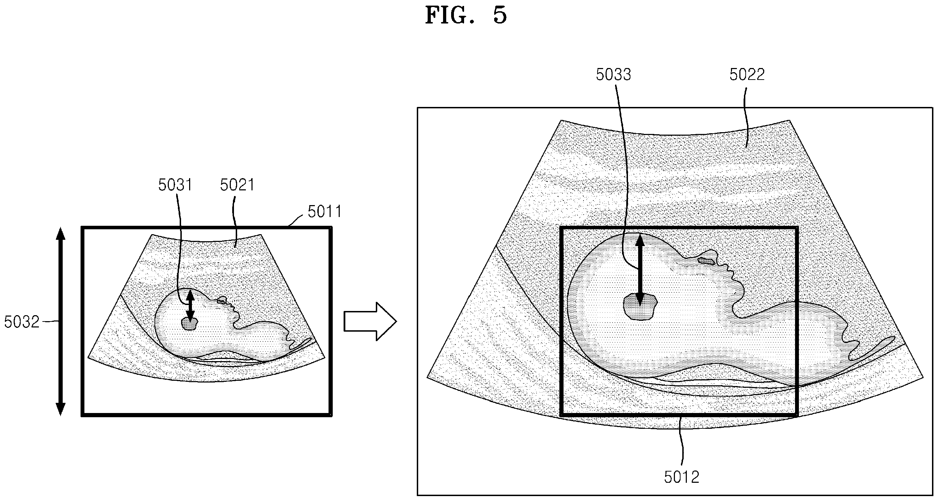

FIG. 5 is a conceptual diagram illustrating a process of enlarging a medical image, which is performed by a medical imaging apparatus, according to an exemplary embodiment of the present invention. Referring to FIGS. 2 and 5, in operation S2020, the display unit may magnify the medical image. According to the present embodiment, the information acquisition unit may acquire information about two landmark points. For example, the information acquisition unit may acquire information about positions of two landmark points respectively corresponding to diencephalon and a head outline on a medical image 5021. The standard view may include a requirement for a ratio between a distance from the position of the diencephalon of a fetus in the medical image 5021 to the head outline therein and an overall size 5032 of a view 5011. The display unit may adjust a distance 5031 to a distance 5033 suitable for the standard view by enlarging or reducing the medical image 5021. Thereafter, the display unit may display a view 5012 of a medical image 5022 based on the medical image 5022 obtained by enlarging or reducing the medical image 5021.

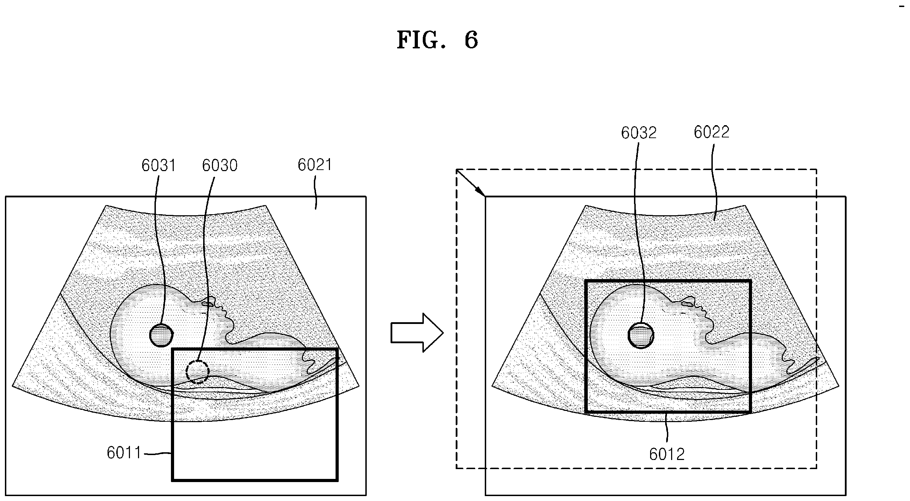

FIG. 6 is a conceptual diagram of a process of shifting a medical image 6021, which is performed by the medical imaging apparatus, according to an exemplary embodiment of the present invention. Referring to FIGS. 2 and 6, in operation S2020, the display unit may move a location of the medical image 6021 that is displayed through a view 6011. According to the present embodiment, the display unit may move the medical image 6021 so as to display a landmark point corresponding to diencephalon 6031 in the medical image 6021 at a position 6030 on the view 6011. As the medical image 6021 moves, the display unit may display diencephalon 6032 on a view 6012 based on a medical image 6022 obtained by moving the medical image 6021.

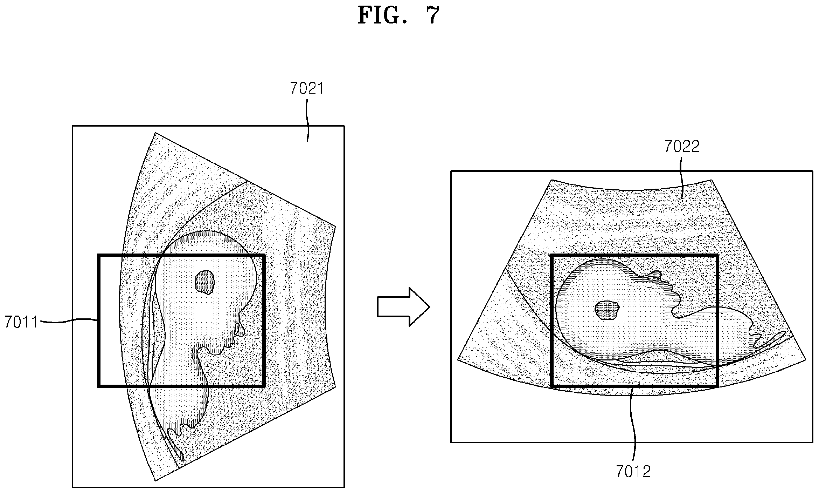

FIG. 7 is a conceptual diagram of a process of rotating a medical image 7021, which is performed by the medical imaging apparatus, according to an exemplary embodiment of the present invention. Referring to FIGS. 2 and 6, in operation S2020, the display unit may rotate the medical image 7021 if a direction of an object in the view 7011 and the medical image 7021 does not comply with requirements defined in the standard view. The display unit may make the direction of the object displayed on the view 7012 suitable for the standard view based on a medical image 7022 obtained by rotating the medical image 7021.

Furthermore, in operation S2020, the display unit may further display a fitness rate between a standard view and a medical image view. The fitness rate may be represented in a qualitative or quantitative manner. For example, the fitness rate may be expressed qualitatively as "excellent", "good", and "poor", or "success" and "failure". As another example, the fitness rate may be expressed quantitatively as "100%/75%/50%/25%/0%" or "5/4/3/2/1" according to the number of requirements that a medical image view displayed on the display unit satisfies among requirements included in the standard view. In another embodiment, if the fitness rate is classified qualitatively as "poor" or "failure" or does not exceed a quantitative thread value, the display unit may display a message indicating readjustment of the medical image view.

In one embodiment, the process may further include an operation (not shown) of selecting a diagnostic mode, which is performed by a diagnostic mode selector of the medical imaging apparatus. In this case, in operation S2020, the display unit may display a view of a medical image of which the geometry has been transformed based on a standard view determined according to a selected diagnostic mode. For example, an ultrasound diagnostic device may select a diagnostic mode for measuring fetal NT from among a plurality of diagnostic modes according to a user's input to the ultrasound diagnostic device. When the diagnostic mode for measuring fetal NT is selected, a display unit of the ultrasound diagnostic device may change a geometry of an ultrasound image into one suitable for a standard view for measuring fetal NT and display an ultrasound image of which the geometry has been changed.

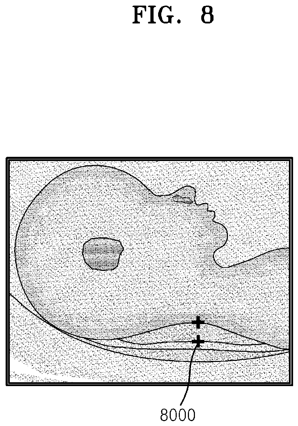

Thereafter, the medical imaging apparatus may measure medical information based on the view displayed on the display unit. FIG. 8 is a conceptual diagram showing measurement of medical information from a medical image displayed in a view, according to an exemplary embodiment of the present invention. Referring to FIG. 8, the medical imaging apparatus may indicate a marker 8000 and measure medical information such as a fetal NT thickness based on the medical image displayed in the view.

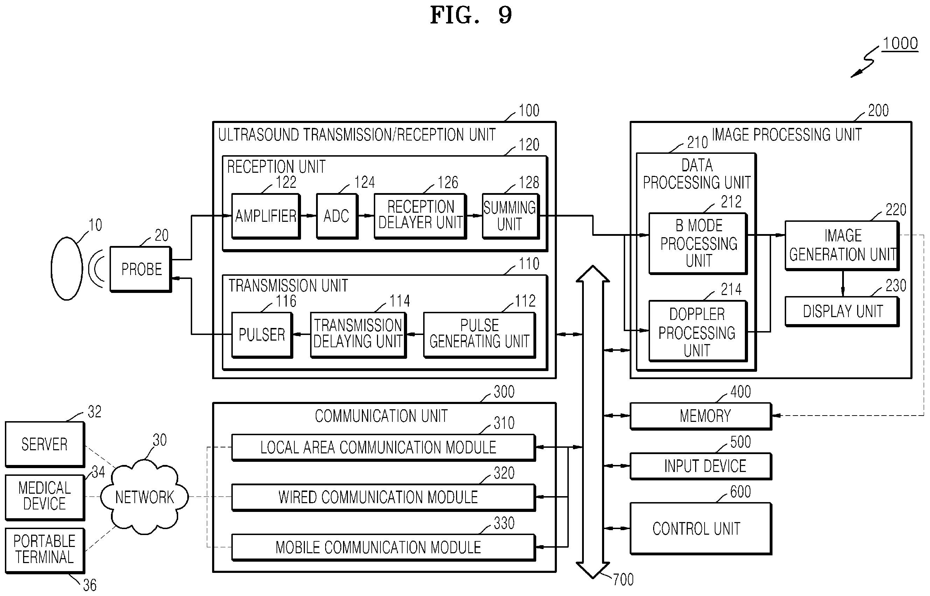

FIG. 9 is a block diagram of a configuration of a medical imaging apparatus implemented as an ultrasound diagnostic device 1000 according to an exemplary embodiment of the present invention.

Referring to FIG. 9, the ultrasound diagnostic device 1000 according to the present embodiment may include a probe 20, an ultrasound transmission/reception unit 100, an image processing unit 200, a communication unit 300, a memory 400, an input device 500, and a control unit 600, and the components may be connected to one another via buses 700.

The ultrasound diagnostic device 1000 may be embodied not only as a cart type device but also as a portable type. Examples of portable ultrasound diagnostic devices may include a PACS viewer, a smartphone, a laptop computer, a personal digital assistant (PDA), and a tablet PC. However, the present invention is not limited thereto.

The probe 20 transmits ultrasound signals to an object 10, based on a driving signal applied by the ultrasound transmission/reception unit 100, and receives echo signals reflected from the object 10. The probe 20 includes a plurality of transducers, and the plurality of transducers oscillate based on electric signals transmitted thereto and generate acoustic energy, that is, ultrasound waves. Furthermore, the probe 20 may be connected to a main body of the ultrasound diagnostic device 1000 by wires or wirelessly. According to embodiments of the present invention, the ultrasound diagnostic device 1000 may include a plurality of probes 20.

A transmission unit 110 supplies a driving signal to the probe 20 and includes a pulse generating unit 112, a transmission delaying unit 114, and a pulser 116. The pulse generating unit 112 generates pulses for forming transmission ultrasound waves based on a predetermined pulse repetition frequency (PRF), and the transmission delaying unit 114 applies a delay time for determining transmission directionality to the pulses. Pulses, to which a delay time is applied, correspond to a plurality of piezoelectric vibrators included in the probe 20, respectively. The pulser 116 applies a driving signal (or a driving pulse) to the probe 20 at a timing corresponding to each pulse to which a delay time is applied.

A reception unit 120 generates ultrasound data by processing echo signals received from the probe 20. The reception unit 120 may include an amplifier 122, an analog-to-digital converter (ADC) 124, a reception delaying unit 126, and a summing unit 128. The amplifier 122 amplifies echo signals in each channel, and the ADC 124 performs analog-to-digital conversion on the amplified echo signals. The reception delaying unit 126 applies delay times for determining reception directionality to the echo signals subjected to the analog-to-digital conversion, and the summing unit 128 generates ultrasound data by summing the echo signals processed by the reception delaying unit 126. According to embodiments of the present invention, the reception unit 120 may not include the amplifier 122. In other words, if the sensitivity of the probe 20 or the capability of the ADC 124 to process bits is enhanced, the amplifier 122 may be omitted.

The image processing unit 200 generates an ultrasound image by scan-converting ultrasound data generated by the ultrasound transmission/reception unit 100 and displays the ultrasound image. In addition, an ultrasound image may include not only a gray-scale ultrasound image obtained by scanning an object in an amplitude (A) mode, a brightness (B) mode, and a motion (M) mode, but also a Doppler image representing a moving object by using a Doppler effect. The Doppler image may include a blood flow Doppler image (also called a color Doppler image) showing a flow of blood, a tissue Doppler image showing movement of tissue, and a spectral Doppler image showing a moving speed of an object as a waveform.

A B mode processing unit 212 extracts B mode components from ultrasound data and processes the B mode components. An image generating unit 220 may generate an ultrasound image indicating signal intensities as brightness based on the extracted B mode components.

Similarly, a Doppler processing unit 214 may extract Doppler components from ultrasound data, and the image generating unit 220 may generate a Doppler image indicating movement of an object as colors or waveforms based on the extracted Doppler components.

The image generating unit 220 according to an embodiment of the present invention may generate a 3D ultrasound image via volume-rendering of volume data and an elasticity image which shows the degree of deformation of the object 10 due to pressure. Furthermore, the image generating unit 220 may display various additional information in an ultrasound image by using text and graphics. In addition, the generated ultrasound image may be stored in the memory 400.

A display unit 230 displays and outputs the generated ultrasound image. The display unit 230 may display and output not only an ultrasound image but also various information processed by the ultrasound diagnostic device 1000 on a screen via a graphical user interface (GUI). In addition, the ultrasound diagnostic device 1000 may include two or more display units 230 according to embodiments of the present invention.

The communication unit 300 is connected to a network 30 by wires or wirelessly and communicates with an external device or a server. The communication unit 300 may exchange data with a hospital server or another medical device in a hospital that is connected via a picture archiving and communications system (PACS). Furthermore, the communication unit 300 may perform data communication according to the digital imaging and communications in medicine (DICOM) standard.

The communication unit 300 may transmit or receive data related to diagnosis of the object 10, e.g., an ultrasound image, ultrasound data, and Doppler data of the object 10, via the network 30. The communication unit 300 may also transmit or receive medical images obtained by other medical devices, such as a CT image, an MR image, and an X-ray image. Furthermore, the communication unit 300 may receive information related to a diagnosis history or a treatment schedule of a patient from a server and utilizes the information for diagnosing the patient. Furthermore, the communication unit 300 may perform data communication with a server or a medical device in a hospital as well as a portable terminal of a doctor or a patient.

The communication unit 300 is connected to the network 30 in a wired or wireless manner and may exchange data with a server 32, a medical device 34, or a portable terminal 36. The communication unit 300 may include at least one component that enables communication with external devices, e.g., a local area communication module 310, a wired communication module 320, and a mobile communication module 330.

The local area communication module 310 is a module for performing local area communication with a device within a predetermined distance. Examples of local area communication technology include a wireless Local Area Network (LAN), Wi-Fi, Bluetooth, ZigBee, Wi-Fi Direct (WFD), Ultra Wideband (UWB), Infrared Data Association (IrDA), Bluetooth Low energy (BLE), and Near Field Communication (NFC), but are not limited thereto.

The wired communication module 320 is a module for performing communication by using an electric signal or an optical signal. Examples of wired communication technology include wired communication technologies using a pair cable, a coaxial cable, an optical fiber cable, and an Ethernet cable.

The mobile communication module 330 transmits or receives wireless signals to or from at least one of a base station, an external terminal, and a server on a mobile communication network. Here, the wireless signals may include voice call signals, video call signals, or various types of data for transmission and reception of text/multimedia messages.

The memory 400 stores various data processed by the ultrasound diagnostic device 1000. For example, the memory 400 may store not only medical data related to the diagnosis of the object 10, such as ultrasound data and ultrasound images that are input or output, but also algorithms or programs that are executed in the ultrasound diagnostic device 1000.

The memory 400 may be embodied as any of various storage media such as a flash memory, a hard disk drive, and an Electrically Erasable Programmable Read-Only Memory (EEPROM). Furthermore, the ultrasound diagnostic device 1000 may utilize a web storage or a cloud server that functions as the memory 400 online.

The input device 500 is a means via which a user inputs data for controlling the ultrasound diagnostic device 1000. The input device 500 may include hardware components, such as a keypad, a mouse, a touch panel, a touch screen, a trackball, and a jog switch. However, the present invention is not limited thereto, and the input device 500 may further include various other input elements such as an electrocardiogram measuring module, a respiration measuring module, a voice recognition sensor, a gesture recognition sensor, a fingerprint recognition sensor, an iris recognition sensor, a depth sensor, a distance sensor, etc.

The control unit 600 may control overall operations of the ultrasound diagnostic device 1000. In other words, the control unit 600 may control operations among the probe 20, the ultrasound transmission/reception unit 100, the image processing unit 200, the communication unit 300, the memory 400, and the input device 500.

All or some of the probe 20, the ultrasound transmission/reception unit 100, the image processing unit 200, the communication unit 300, the memory 400, the input device 500, and the control unit 600 may be operated by software modules. However, the present invention is not limited thereto, and some of the above components may be operated by hardware modules. Furthermore, at least one of the ultrasound transmission/reception unit 100, the image processing unit 200, and the communication unit 300 may be included in the control unit 600, but are not limited thereto.

According to one embodiment, the information acquisition unit 1010 shown in FIG. 1 may be constituted by a combination of the memory 400, the input device 500, and the control unit 600 shown in FIG. 9, Furthermore, the display unit 1020 shown in FIG. 1 may be constituted by the image processing unit 200 shown in FIG. 9. However, the present invention is not limited thereto.

Exemplary embodiments of the present invention may be implemented through computer-readable recording media having recorded thereon computer-executable instructions such as program modules that are executed by a computer. Computer-readable recording media may be any available media that can be accessed by a computer and include both volatile and nonvolatile media and both detachable and non-detachable media. Furthermore, the computer-readable media may include computer storage media and communication media. The computer storage media include both volatile and nonvolatile and both detachable and non-detachable media implemented by any method or technique for storing information such as computer-readable instructions, data structures, program modules or other data. The communication media typically embody computer-readable instructions, data structures, program modules, other data of a modulated data signal, or other transmission mechanism, and they include any information transmission media. For example, the computer storage media may be implemented as ROM, random access memory (RAM), flash memory, a compact disc (CD), a digital versatile disc (DVD), a magnetic disk, or a magnetic tape.

While one or more embodiments of the present invention have been described with reference to the figures, it will be understood by those of ordinary skill in the art that various changes in form and details may be made therein without departing from the scope of the present invention as defined by the following claims. Thus, it should be understood that the exemplary embodiments described therein should be considered in a descriptive sense only and not for purposes of limitation.

## Claims

1. A method of displaying an ultrasound image using a medical imaging apparatus, the method comprising:
acquiring seed information including position information of a body structure and outline of the body structure from a user input;
acquiring information related to a plurality of landmark points indicating a feature related to the body structure in the ultrasound image based on the seed information, wherein the information related to a plurality of landmark points includes at least one of a position of the plurality of landmark points, a distance between the plurality of landmark points and an area related to the body structure corresponding to the plurality of landmark points;
shifting a view of the ultrasound image so as to locate a first landmark point at a center region of the medical image;
receiving a user input for selecting a diagnostic mode from among a plurality of diagnostic modes of the medical imaging apparatus;
performing at least one of enlargement, reduction, rotation, and translation on the ultrasound image so that a ratio of a distance between the first landmark point and a second landmark point to an overall size of the view of the ultrasound image satisfies a required ratio in a standard view, wherein the standard view includes information about at least one of a position, a size, a direction of the body structure contained in the ultrasound image, and the required ratio; and
displaying a view of the ultrasound image,
wherein the requirements included in the standard view are determined according to the selected diagnostic mode by the user input.

2. The method of claim 1, wherein in the displaying of the view of the ultrasound image, a fitness rate between the standard view and the view of the ultrasound image is displayed.

3. The method of claim 1, wherein the view of the ultrasound image that satisfies the standard view includes an image obtained by shifting the ultrasound image so that the plurality of landmark points is located at a position included in the standard view.

4. The method of claim 1, wherein the view of the ultrasound image that satisfies the standard view includes an image obtained by rotating the ultrasound image at an angle determined based on the plurality of landmark points and the standard view.

5. The method of claim 1, wherein the acquiring seed information comprises acquiring the seed information related to the ultrasound image which is information related to a position of the body structure in the ultrasound image for capturing a middle sagittal image,
wherein in the acquiring of the information about the plurality of landmark points, the information about the plurality of landmark points is acquired based on the seed information.

6. A medical imaging apparatus (1000) comprising:
an information acquisition unit (1010) configured to acquire seed information including position information of a body structure and outline of the body structure from a user input, and information related to a plurality of landmark points indicating a feature related to the body structure in the ultrasound image based on the seed information, wherein the information related to a plurality of landmark points includes at least one of a position of the plurality of landmark points, a distance between the plurality of landmark points and an area related to the body structure corresponding to the plurality of landmark points;
a diagnostic mode selector configured to receive a user input for selecting a diagnostic mode from among a plurality of diagnostic modes of the medical imaging apparatus; and
a display unit (1020) configured to shift a view of the ultrasound image so as to locate a first landmark point at a center region of the ultrasound image, and perform at least one of enlargement, reduction, rotation, and translation on the ultrasound image so that a ratio of a distance between the first landmark point and a second landmark point to an overall size of the view of the ultrasound image satisfies a required ratio included in a standard view, wherein the standard view includes information about at least one of a position, a size, a direction of the body structure contained in the ultrasound image, and the required ratio, and to display a view of the ultrasound image,
wherein the requirements included in the standard view are determined according to the selected diagnostic mode by the diagnostic mode selector.

7. The apparatus (1000) of claim 6, wherein the information acquisition unit (1010) is further configured to acquire the seed information related to the ultrasound image which is information related to a position of the body structure in the ultrasound image for capturing a middle sagittal image and information about the plurality of landmark points based on the seed information.

## Patentansprüche

1. Verfahren zum Anzeigen eines Ultraschallbilds unter Verwendung einer medizinischen Bildgebungsvorrichtung, wobei das Verfahren Folgendes aufweist:
Erlangen von Seedinformationen, die Positionsinformationen einer Körperstruktur und der Kontur der Körperstruktur enthalten, aus einer Benutzereingabe;
Erlangen von Informationen bezüglich einer Vielzahl von Orientierungspunkten, die ein sich auf die Körperstruktur beziehendes Merkmal anzeigen, in dem Ultraschallbild, basierend auf den Seedinformationen, wobei die Informationen bezüglich einer Vielzahl von Orientierungspunkten wenigstens eines einer Position der Vielzahl von Orientierungspunkten, eines Abstands zwischen der Vielzahl von Orientierungspunkten und eines Bereichs beinhalten, der sich auf die Körperstruktur bezieht, die der Vielzahl von Orientierungspunkten entspricht;
Verschieben einer Ansicht des Ultraschallbilds, um einen ersten Orientierungspunkt in einem mittleren Bereich des medizinischen Bilds anzuordnen;
Empfangen einer Benutzereingabe zum Auswählen eines Diagnosemodus aus einer Vielzahl von Diagnosemodi der medizinischen Bildgebungsvorrichtung;
Durchführen wenigstens eines von Vergrößerung, Verkleinerung, Rotation und Verschiebung an dem Ultraschallbild, so dass ein Verhältnis eines Abstands zwischen dem ersten Orientierungspunkt und einem zweiten Orientierungspunkt zu einer Gesamtgröße der Ansicht des Ultraschallbilds ein erforderliches Verhältnis in einer Standardansicht erfüllt, wobei die Standardansicht Informationen über wenigstens eines einer Position, einer Größe, einer Richtung der Körperstruktur, die in dem Ultraschallbild enthalten ist, und des erforderlichen Verhältnisses beinhaltet; und
Anzeigen einer Ansicht des Ultraschallbilds;
wobei die in der Standardansicht enthaltenen Erfordernisse gemäß des ausgewählten Diagnosemodus durch die Benutzereingabe ermittelt werden.

2. Verfahren nach Anspruch 1, wobei bei dem Anzeigen der Ansicht des Ultraschallbilds ein Eignungsverhältnis zwischen der Standardansicht und der Ansicht des Ultraschallbilds angezeigt wird.

3. Verfahren nach Anspruch 1, wobei die Ansicht des Ultraschallbilds, die die Standardansicht erfüllt, ein Bild beinhaltet, das durch derartiges Verschieben des Ultraschallbilds erhalten wird, dass die Vielzahl von Orientierungspunkten an einer Position angeordnet ist, die in der Standardansicht enthalten ist.

4. Verfahren nach Anspruch 1, wobei die Ansicht des Ultraschallbilds, die die Standardansicht erfüllt, ein Bild beinhaltet, das durch Rotieren des Ultraschallbilds um einen Winkel erhalten wird, der basierend auf der Vielzahl von Orientierungspunkten und der Standardansicht ermittelt wird.

5. Verfahren nach Anspruch 1, wobei das Erlangen der Seedinformationen das Erlangen der Seedinformationen bezüglich des Ultraschallbilds beinhaltet, wobei es sich um Informationen bezüglich einer Position der Körperstruktur in dem Ultraschallbild zum Erfassen eines mittleren Sagittalbilds ist,
wobei bei dem Erlangen der Informationen bezüglich der Vielzahl von Orientierungspunkten die Informationen bezüglich der Vielzahl von Orientierungspunkten basierend auf den Seedinformationen erlangt wird.

6. Medizinische Bildgebungsvorrichtung (1000), welche Folgendes aufweist:
eine Informationserlangungseinheit (1010), die dafür vorgesehen ist, Seedinformationen einschließlich Positionsinformationen einer Körperstruktur und einer Kontur der Körperstruktur aus einer Benutzereingabe, und Informationen bezüglich einer Vielzahl von Orientierungspunkten, die ein Merkmal bezüglich der Körperstruktur in dem Ultraschallbild basierend auf den Saatinformationen anzeigen zu erlangen, wobei die Informationen bezüglich einer Vielzahl von Orientierungspunkten wenigstens eines einer Position der Vielzahl von Orientierungspunkten, eines Abstands zwischen der Vielzahl von Orientierungspunkten und eines Bereichs bezüglich der Körperstruktur, die der Vielzahl von Orientierungspunkten entspricht, beinhaltet;
eine Diagnosemodusauswahleinrichtung, die dafür vorgesehen ist, eine Benutzereingabe zum Auswählen eines Diagnosemodus aus einer Vielzahl von Diagnosemodi der medizinischen Bildgebungsvorrichtung zu empfangen; und
eine Anzeigeeinrichtung (1020), die dafür vorgesehen ist, eine Ansicht des Ultraschallbilds so zu verschieben, dass ein erster Orientierungspunkt in einem mittleren Bereich des Ultraschallbilds angeordnet ist, und wenigstens eines einer Vergrößerung, einer Verkleinerung, einer Rotation und einer Verschiebung an dem Ultraschallbild durchzuführen, so dass ein Verhältnis eines Abstands zwischen dem ersten Orientierungspunkt und einem zweiten Orientierungspunkt zu einer Gesamtgröße der Ansicht des Ultraschallbilds ein erforderliches Verhältnis erfüllt, das in einer Standardansicht enthalten ist, wobei die Standardansicht Informationen bezüglich wenigstens eines einer Position, einer Größe, einer Richtung der Körperstruktur, die in dem Ultraschallbild enthalten ist, und des erforderlichen Verhältnisses beinhaltet, und um eine Ansicht des Ultraschallbilds anzuzeigen,
wobei die Erfordernisse, die in der Standardansicht enthalten sind, gemäß des ausgewählten Diagnosemodus durch die Diagnosemodusauswähleinheit ermittelt werden.

7. Vorrichtung (1000) nach Anspruch 6, wobei die Informationserlangungseinheit (1010) des Weiteren dafür vorgesehen ist, die Seedinformationen bezüglich des Ultraschallbilds, die Informationen bezüglich einer Position der Körperstruktur in dem Ultraschallbild zum Erfassen eines mittleren Sagittalbilds und Informationen bezüglich der Vielzahl von Orientierungspunkten basierend auf den Saatinformationen zu erlangen.

## Revendications

1. Procédé d'affichage d'une image ultrasonique, en utilisant un appareil d'imagerie médicale, le procédé comportant :
l'acquisition d'informations de source incluant une information de position d'une structure de corps et le profil de la structure de corps au moyen d'une entrée d'utilisateur ;
l'acquisition d'information relative à une pluralité de points de repères indiquant des éléments relatifs à la structure de corps dans l'image ultrasonique basée sur les informations de source, dans lequel l'information relative à une pluralité de points de repères inclut au moins une des positions de la pluralité de points de repères, une distance entre la pluralité de points de repères et une zone relative à la structure de corps correspondant à la pluralité de points de repères ;
le déplacement d'une vue de l'image ultrasonique de manière à localiser un premier point de repère dans une région centrale de l'image médicale ;
la réception d'une entrée d'utilisateur pour sélectionner un mode de diagnostic parmi une pluralité de modes de diagnostics de l'appareil d'imagerie médicale ;
effectuer au moins une opération d'agrandissement, de réduction, de rotation et de translation sur l'image ultrasonique, de sorte qu'un rapport d'une distance entre le premier point de repère et un second point de repère à une taille globale de la vue de l'image ultrasonique, satisfait un rapport requis dans une vue standard, dans lequel la vue standard inclut des informations concernant au moins une position, une taille, une direction de la structure du corps contenue dans l'image ultrasonique, et le rapport requis; et
l'affichage d'une vue de l'image ultrasonique,
dans laquelle les exigences inclues dans la vue standard sont déterminées selon le mode de diagnostic sélectionné par l'entrée de l'utilisateur.

2. Procédé selon la revendication 1, dans lequel l'affichage de la vue de l'image ultrasonique, un taux de forme physique entre la vue standard et la vue de l'image ultrasonique sont affichés.

3. Procédé selon la revendication 1, dans lequel la vue de l'image ultrasonique qui satisfait la vue standard inclut une image obtenue en déplaçant l'image ultrasonique de sorte que la pluralité de points de repères soit localisée dans une position incluse dans l'image standard.

4. Procédé selon la revendication 1, dans lequel la vue de l'image ultrasonique qui satisfait la vue standard inclut une image obtenue en tournant l'image ultrasonique d'un angle déterminé basé sur la pluralité de points de repères et la vue standard.

5. Procédé selon la revendication 1, dans lequel l'acquisition de l'information de source comprend l'acquisition d'informations de source relatives à l'image ultrasonique qui est une information relative à une position de la structure du corps sur l'image ultrasonique pour capturer une image sagittale médiane,
dans lequel, dans l'acquisition des informations concernant la pluralité des points de repères, les informations relatives à la pluralité de points de repères sont acquises sur la base des informations de source.

6. Appareil d'imagerie médicale (1000) comprenant :
une unité d'acquisition d'informations (1010) configurée pour acquérir des informations de source incluant une information de position d'une structure de corps et le profil de la structure de corps au moyen d'une entrée d'utilisateur; et des informations relatives à une pluralité de points de repères indiquant des éléments relatifs à la structure de corps dans l'image ultrasonique basée sur les informations de source, dans lequel, les informations relatives à une pluralité de points de repères inclut au moins une des positions de la pluralité de points de repères, une distance entre la pluralité de points de repères et une zone relative à la structure de corps correspondant à la pluralité de points de repères;
un sélecteur de mode de diagnostic configuré pour recevoir une entrée d'utilisateur pour sélectionner un mode de diagnostic parmi une pluralité de modes de diagnostics de l'appareil d'imagerie médicale; et
une unité d'affichage (1020) configurée pour déplacer une vue de l'image médicale de manière à situer un premier point de repère dans une région centrale de l'image ultrasonique, et pour effectuer au moins une opération d'agrandissement, de réduction, de rotation et de translation sur l'image ultrasonique, de sorte qu'un rapport d'une distance entre le premier point de repère et un second point de repère à une taille globale de la vue de l'image ultrasonique, satisfait un rapport requis dans une vue standard, dans lequel la vue standard inclut des informations concernant au moins une position, une taille, une direction de la structure du corps contenue dans l'image ultrasonique, et le rapport requis; et afficher une vue de l'image ultrasonique,
dans laquelle les exigences inclues dans la vue standard sont déterminées selon le mode de diagnostic sélectionné par le sélecteur de mode de diagnostic.

7. Appareil (1000) selon la revendication 6, dans lequel l'unité d'acquisition d'informations (1010) est en outre configurée pour acquérir les informations de source relatives à l'image ultrasonique, qui sont des informations relatives à la position de la structure du corps dans l'image ultrasonique pour capter une image sagittale médiane et des informations relatives à la pluralité des points de repères basés sur les informations de source.
